# EUROPEAN PATENT SPECIFICATION

(11) **EP 1 549 950 B1**
(45) Date of publication and mention of the grant of the patent: **11.02.2009**
(21) Application number: 03761006.0
(22) Date of filing: 25.06.2003
(51) Int. Cl.: G01N 33/50, A61P 3/10

(54) **METHODS FOR IDENTIFYING GLUCOSE UPTAKE MODULATORS**
VERFAHREN ZUR IDENTIFIZIERUNG VON MODULATOREN DER GLUCOSEAUFNAHME
PROCEDES PERMETTANT L'IDENTIFICATION DE MODULATEURS DE L'ASSIMILATION DU GLUCOSE

(30) Priority: 25.06.2002 US 391989 P; 28.06.2002 SE 0202017
(43) Date of publication of application: 06.07.2005
(73) Proprietor: Biovitrum AB (publ), 112 76 Stockholm (SE)
(72) Inventor: KATZ, Abram, S-162 45 Vällingby (SE); WESTERBLAD, Håkan, S-131 42 Nacka (SE)
(74) Representative: Höglund, Lars
(86) International application number: PCT/SE2003/001103
(87) International publication number: WO 2004/001410

(56) References cited:
- WO-A1-98/08979
- PAVEL SHASHKIN ET AL.: 'Effects of CGS 9343B (a putative calmodulin antagonist) on isolated skeletal muscle' THE JOURNAL OF BIOLOGICAL CHEMISTRY vol. 270, no. 43, October 1995, pages 25613 - 25618, XP002974400
- BRUTON JOSEPH D. ET AL.: 'Insulin increases near-membrane but not global Ca2+ in isolated skeletal muscle' PROC. NATL. ACAD. SCI. USA vol. 96, March 1999, pages 3281 - 3286, XP002975001
- NAGOMI KUREBAYASHI ET AL.: 'Depletion of Ca2+ in the sarcoplasmic reticulum stimulates Ca2+ entry into mouse skeletal muscle fibres' JOURNAL OF PHYSIOLOGY vol. 533, no. 1, 2001, pages 185 - 199, XP002975002

## Description

### TECHNICAL FIELD

The present invention relates to methods for identifying agents modulating store-operated Ca²⁺ entry and thereby modulating glucose uptake in mammalian cells. Such compounds may be useful for the treatment of diseases connected with reduced glucose uptake, such as type 2 diabetes.

### BACKGROUND ART

Skeletal muscle is quantitatively the most important site of insulin-mediated glucose uptake and this uptake is impaired in patients with type 2 diabetes. Understanding of how insulin increases glucose uptake in skeletal muscle has increased during recent years, but many unresolved questions still remain (Ryder et al. (2001) Acta Physiol Scand 171:249-257). Whether insulin action involves increases in intracellular Ca²⁺ has been debated (Klip and Ramlal (1987) J Biol. Chem. 262:9141-9146; Youn et al. (1994) Am J Physiol 267:R888-R894). Most studies have not been able to detect insulin-mediated increases in free intracellular Ca²⁺ concentration (Klip and Ramlal (1987) J Biol. Chem. 262:9141-9146; Cheung et al. (1987) Am J Physiol 252:C163-C172; Kelly et al. (1989) J Biol. Chem. 264:12754-12757). Furthermore, incubation of isolated rodent skeletal muscles in the presence of nominal [Ca²⁺] in the incubation medium does not consistently alter insulin-mediated glucose transport (Clausen (1980) Cell Calcium 1:311-325). Indeed a large generalized increase of intracellular Ca²⁺ concentration (e.g. by using Ca²⁺ ionophores) results, if anything, in decreased insulin-mediated glucose uptake (Draznin et al. (1987) J. Biol. Chem. 262:14385-14388; Lee et al. (1995) Am. J. Physiol. 268:R997-R1002).

Recent studies suggest that Ca²⁺ and the Ca²⁺-binding protein calmodulin are involved in insulin-stimulated glucose transport in skeletal muscle (Shashkin et al. (1995) J Biol Chem 270:25613-25618; Brozinick et al. (1999) Biochem J 339:533-540; Bruton et al. (1999) Proc Natl Acad Sci U S A 96:3281-3286; Bruton et al. (2001) Acta Physiol Scand 171:259-265) as well as in adipocytes (Whitehead et al. (2001) J Biol Chem 276:27816-27824). In skeletal muscle the most direct evidence to support this view lies in the observation that insulin increases the near-membrane free [Ca²⁺] ([Ca²⁺]ₘₑₘ) and that this increase is attenuated by interventions that inhibit insulin-mediated glucose transport (Bruton et al. (1999) Proc Natl Acad Sci U S A 96:3281-3286). The insulin-induced increase in [Ca²⁺]ₘₑₘ was inhibited by L-type Ca²⁺ channel blockers. However, the involvement of L-type Ca²⁺ channels remains unclear since these channels are normally activated by depolarization and insulin application causes slight hyperpolarization (Bruton et al., *supra*).

Nucleic acids coding for calcium channel polypeptides are disclosed e.g. in WO 00/40614. Calcium channels are membrane-spanning, multi-subunit proteins that facilitate the controlled transport ("flux") of Ca²⁺ ions into and out of cells. In general, "excitable" cells, such as neurons and skeletal muscle cells, possess voltage-dependent calcium channels. In "non-excitable" cells, calcium influx is thought to occur predominantly in response to stimuli which cause the release of calcium from intracellular stores. This process, termed "store operated" calcium influx, is not well understood.

Recent studies suggest the existence of "store-operated calcium" (SOC), "calcium-release activated calcium" (CRAC) or "store-mediated" Ca²⁺ entry (SMCE) channels in skeletal muscle (Putney et al. (2001) J Cell Sci 114:2223-2229; Kurebayashi and Ogawa (2001) J Physiol 533:185-199). However, the physiological significance of this process is unclear in skeletal muscle as well as in most other excitable cells.

### BRIEF DESCRIPTION OF THE DRAWINGS

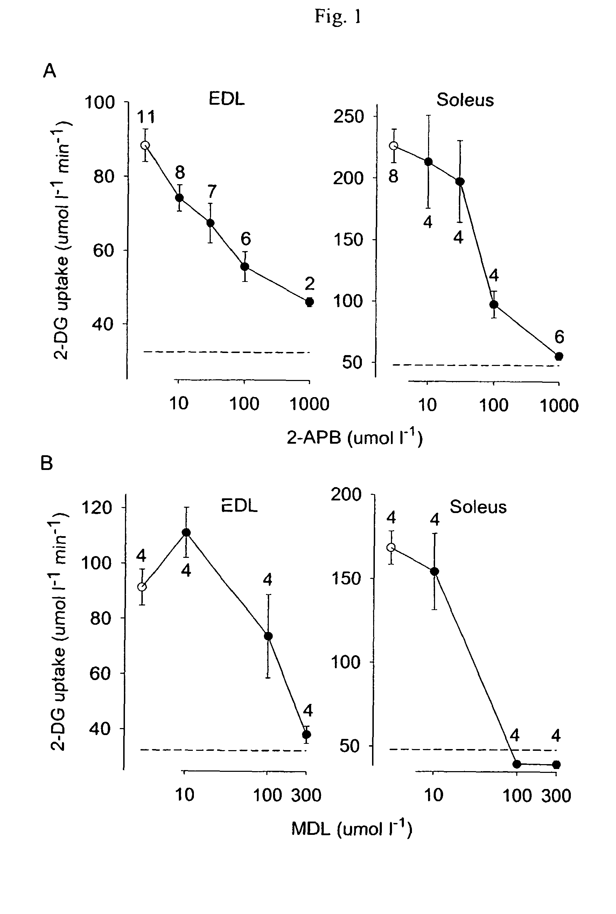

Figs. 1A-1B are graphs demonstrating that pharmacological inhibition of "store-mediated" Ca²⁺ entry (SMCE) decreases insulin-stimulated 2-deoxyglucose (2-DG) uptake. 2-DG uptake during insulin (10 milliunits ml⁻¹) exposure in EDL (left) and soleus (right) muscles is plotted *vs.* the concentration of diphenylboric acid 2-aminoethyl ester (also called 2-aminoethoxydiphenyl borate) (2-APB) (*A*) or cis-*N*-(2-phenylcyclopentyl)azacyclotridec-1-en-2-amine (also called MDL-12,330A) (MDL) (*B*), inhibitors of SMCE. Unfilled circles denote the rate of insulin-stimulated uptake in the absence of SMCE inhibitors. Values are presented as mean ± SEM and the number of muscles tested is indicated for each point. Dashed lines indicate the mean basal rate of 2-DG uptake (i.e. without insulin and SMCE inhibitors).

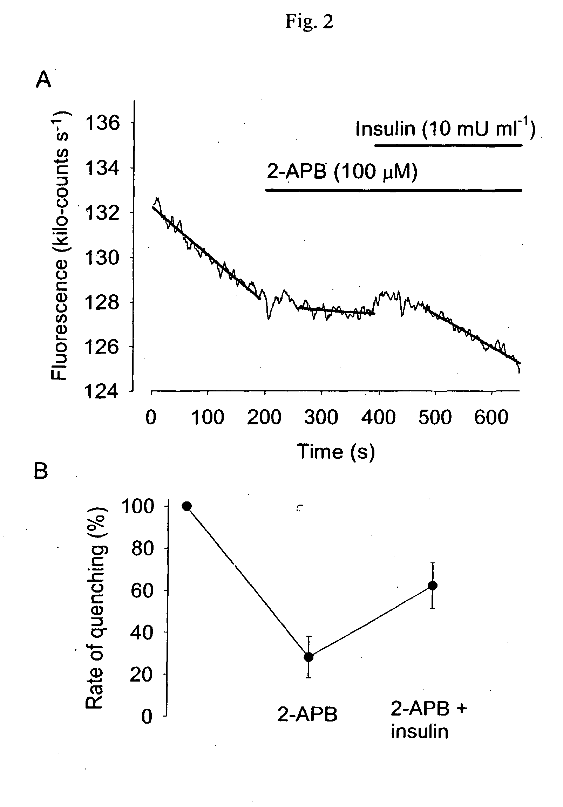

Figs. 2A-2B are graphs demonstrating that the application of 2-APB decreases the rate of Mn²⁺ quenching of fura-2 fluorescence intensity and the decrease is partially reversed by insulin. 2*A*: Original record of fura-2 fluorescence from a single muscle fiber. Straight lines represent linear fits that were used to measure the rate of decline during periods with a stable decrease rate. 2*B*: Mean data (± SEM; n = 6) of the rate of quenching during exposure to 2-APB and 2-APB + insulin. The rate of quenching before application of 2-APB was set to 100% in each fiber.

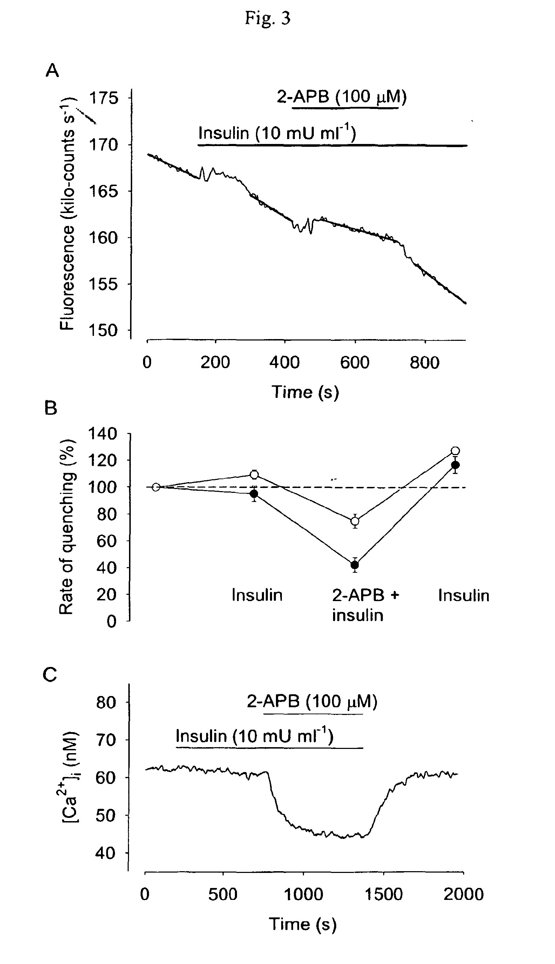

Figs. 3A-3b are graphs demonstrating that the rate of Mn²⁺ quenching of fura-2 fluorescence intensity is decreased by application of 2-APB in the presence of insulin and increased after the subsequent washout of 2-APB. 3*A*: Original record from a single muscle fiber; straight lines represent linear fits. 3*B*: Mean data (± SEM) of the relative rate of Mn²⁺ quenching in the type of experiment shown in A performed in 5.5 mmol 1⁻¹ (filled circles; n = 6) and 0 mmol 1⁻¹ (unfilled circles; n = 5) glucose in the bath solution. The rate of quenching before application of insulin and 2-APB was set to 100% in each experiment (dashed line). 3C: Application of insulin had no effect on global free myoplasmic [Ca²⁺] ([Ca²⁺]ᵢ) (measured with indo-1), whereas the subsequent application of 2-APB caused a marked decrease. Record is representative of four experiments.

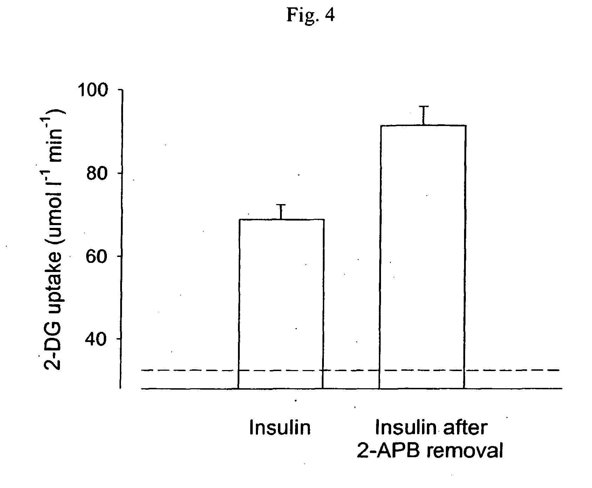

Fig. 4 is a graph depicting increased 2-DG uptake after 2-APB removal in the continued presence of insulin (P<0.01). Mean data (± SEM) from 8 muscles in each group. Dashed line indicates 2-DG uptake under basal conditions (i.e. with out insulin).

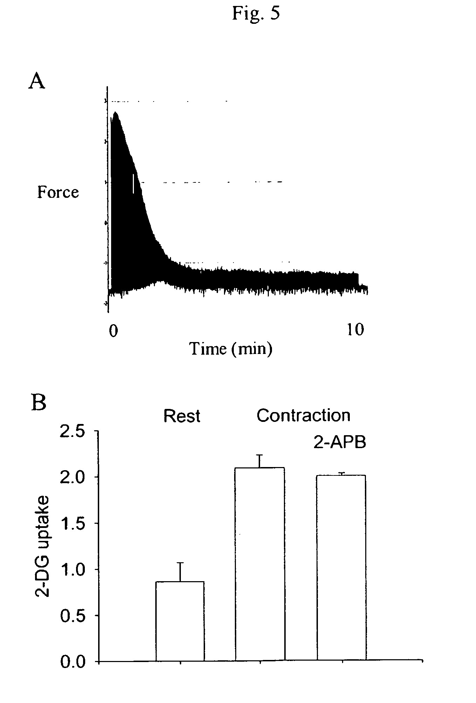

Figs. 5A-5B are graphs demonstrating that 2-APB does not inhibit contraction-induced 2-DG uptake. 5A. Representative force record from an EDL muscle exposed to repeated tetanic stimulation (50 Hz, 100 ms duration, 2 contractions/s). 5B. Mean data (± SEM; n = 5) of 2-DG uptake after the period of repeated contractions in the absence and presence of 2-APB and in rested control muscles.

### DISCLOSURE OF THE INVENTION

It has surprisingly been found that "store-mediated" Ca²⁺ entry (SMCE) modulates insulin-stimulated glucose uptake in skeletal muscle. Pharmacological inhibition of SMCE by 2-APB or MDL resulted in a dose-dependent decrease of insulin-mediated 2-deoxyglucose uptake in isolated mouse fast-twitch and slow-twitch skeletal muscles. Neither of these compounds affected basal 2-deoxyglucose uptake. The rate of Mn²⁺ quenching of fura-2 fluorescence intensity was used as an indirect measure of SMCE. Application of 2-APB reduced the rate of Mn²⁺ quenching by about 70% and this decrease was partially reversed by insulin. The effect of 2-APB on Mn²⁺ quenching was larger in the presence than in the absence of extracellular glucose. Removal of 2-APB in the continued presence of insulin increased the rate of Mn²⁺ quenching by about 20% as compared to insulin application on its own. This increase in Mn²⁺ quenching was associated with ∼ a 30% increase in insulin-mediated 2-deoxyglucose uptake. 2-APB exposure had no effect on contraction-mediated 2-deoxyglucose uptake. These results support a physiological role of SMCE and Ca²⁺ alterations in insulin action in skeletal muscle, where an increase in Ca²⁺ entry results in an increased insulin-mediated glucose uptake. Thus, SMCE could be a suitable target for future pharmacological treatment of type 2 diabetes.

Consequently, in a first aspect this invention provides a method for identifying an agent that modulates glucose uptake in a mammalian cell, the method comprising: contacting a mammalian cell with a candidate agent; determining whether the candidate agent modulates store-mediated Ca²⁺ entry (SMCE) into the cell; and determining whether the candidate agent modulates glucose uptake in the cell. The method can include, for example, determining whether the candidate agent increases SMCE into the cell. The method can also include determining whether the candidate agent increases glucose uptake in the mammalian cell.

Preferably, the agent increases the open probability of SMCE channels, and thereby the entry of Ca²⁺ and glucose uptake in the cell. Such facilitators of SMCE may be useful for the treatment of medical conditions involving reduced glucose uptake, such as type 2 diabetes. Candidate agents encompass numerous chemical classes, although typically they are organic compounds. Candidate agents can be obtained from a wide variety of sources including libraries of synthetic or natural compounds. A candidate agent can contain, for example, a peptide, peptidomimetic, amino acid, amino acid analog, polynucleotide, polynucleotide analog, nucleotide, nucleotide analog, or other small molecule.

Examples of SMCE-regulating polypeptides, referred to as "SOC/CRAC calcium channel polypeptides", are disclosed in WO 00/40614. Specifically, the amino acid sequences of human SOC/CRAC calcium channel polypeptides are disclosed as SEQ ID NO: 28, 30 and 32 in WO 00/40614.

Various assays can be used to screen for compounds that modulate the function, as agonists or antagonists, of calcium channels. Several examples of such screening assays have been described; see e.g. U.S. Patent No. 5,643,750.

### Fura 2 assay

Human calcium channel expressing animal cells can be employed in the presence of calcium-sensitive, fluorescent dyes (for example fura-2, fluo-3 or indo-1) for measurements of the intracellular calcium concentration after opening and blocking of the calcium channels (See e.g. Rosario et al. (1989) Neurosci. 29: 735-747). The change in the intracellular calcium concentration can in this case be measured by fluorimetry (spectrophotometry). For example, cultured cells can be loaded with fura-2. MnCl₂ is then added while measuring the rate of quenching (decline) of fura-2 fluorescence. A drug candidate is then added and any change in the rate of Mn²⁺ quenching is determined (cf. Example 5).

### Assay based on genetically-encoded fluorescent Ca²⁺ indicators

In this assay Ca²⁺ is measured with a cameleon, i.e. a fluorescent Ca²⁺ indicator protein. A cameleon consists of a cyan fluorescent protein, calmodulin (CaM), a glycylglycine linker, the CaM-binding domain of myosin light chain kinase, and a yellow fluorescent protein. Ca²⁺ binding to CaM results in a change of the conformation of this molecule and hence altered fluorescence.

### Receptor binding assay

Cultured cells transformed with human calcium channels can be cultivated and employed for the preparation of membranes. These membrane preparations can be employed in binding studies with various classes of radioactively labeled substances for screening novel ligands (competitive assay). Examples of known calcium channel binding substances are: phenylalkylamines; benzothiazepines; dihydropyridines; bisphenylbutylpiperidines; and omega conotoxins.

### Calcium-45 flux assay

Calcium entry through membranes of cultured cells that have been transformed with human calcium channels can be altered and measured using radioactively labeled calcium (⁴⁵Ca) (See e.g. Messing et al. (1985) J. Pharmacology and Exp. Therapeutics 235: 407-411) and thereby employed for the functional testing/screening of calcium channel antagonists or agonists.

### Electrophysiology

The calcium currents generated SMCE can be measured electrophysiologically (See e.g. Carbone et al. (1990) Pflugers Arch. 416: 170-179). The effect of potential calcium channel antagonists or agonists can be physically measured and pharmacologically characterized directly in human calcium channels using the recombinant animal cell lines.

### Indirect methods of measurement

Many cellular processes are controlled by the intracellular calcium ion concentration (for example receptor-mediated signal transmission, various enzyme reactions, such as, for example, phosphorylation, dephosphorylations, neurotransmitter release, Ca-dependent gene regulation etc). Some of these biochemical reactions can be measured using a specific assay. It is thus possible in a recombinant calcium channel-expressing cell system to detect indirectly (physiologically) the effect of calcium channel modulators on calcium-dependent cellular processes (See e.g. Zernig et al. (1986) Eur. J. Pharmacol. 128: 221-229).

It is additionally possible by modifications introduced by targeted mutagenesis, such as, for example, point mutations, insertions, deletions, replacement of DNA segments of various calcium channel subtypes, to detect direct effects on physiological processes (See e.g. Yool and Schwarz (1991) Nature 349: 700-704).

The term "treatment" means any treatment of a diseases in a mammal, including: (i) preventing the disease, i.e. causing the clinical symptoms of the disease not to develop; (ii) inhibiting the disease, i.e. arresting the development of clinical symptoms; and/or (iii) relieving the disease, i.e. causing the regression of clinical symptoms. The term "effective amount" means a dosage sufficient to provide treatment for the disease state being treated. This will vary depending on the patient, the disease and the treatment being effected.

Throughout this description the terms "standard protocols" and "standard procedures," when used in the context of molecular biology techniques, are to be understood as protocols and procedures found in an ordinary laboratory manual such as: Current Protocols in Molecular Biology, editors F. Ausubel et al., John Wiley and Sons, Inc. 1994, or Sambrook, J., Fritsch, E.F. and Maniatis, T., Molecular Cloning: A laboratory manual, 2nd Ed., Cold Spring Harbor Laboratory Press, Cold Spring Harbor, NY 1989.

Below, the invention is described in the appended examples, which are intended to illustrate the invention, without limiting the scope of protection.

### RESEARCH DESIGN AND METHODS

Adult male mice (NMRI strain) were housed at room temperature and fed *ad libitum.* Animals were killed by rapid neck disarticulation and muscles removed. Extensor digitorum longus (EDL; mainly fast-twitch fibers)) and soleus (mainly slow-twitch fibers) muscles were used for measurements of 2-deoxyglucose (2-DG) uptake. Flexor digitorum brevis (FDB, mainly fast-twitch fibers) muscles were used in single fiber experiments. All procedures were approved by the local ethics committee.

2-DG uptake was measured essentially as described elsewhere (Shashkin et al. (1995) J Biol Chem 270:25613-25618; Le Marchand-Brustel et al. (1979) J Clin Invest 64:1505-1515). Briefly, muscles were incubated at 35°C in Krebs bicarbonate buffer containing 2 mmol 1⁻¹ pyruvate (no glucose) +/- an antagonist of SMCE, either 2-APB, a relatively specific inhibitor of SMCE that appears to act directly on SMCE channels (Braun et al. (2001) J Biol Chem 276:1063-1070; Broad et al. (2001) J Biol Chem 276:15945-15952), or MDL, another inhibitor of SMCE with different modes of action as compared to 2-APB (van Rossum et al. (2000) J Biol Chem 275:28562-28568). In other experiments muscles were incubated in 2,5-di(*tert*-butyl)-1,4-benzohydroquinone (TBQ), an inhibitor of SR Ca²⁺ uptake (Westerblad et al. (1994) J Physiol 474:291-301). In some experiments insulin was added (10 milliunits ml⁻¹) after the initial 30 min incubation period. After an additional 30 min, 2-DG (1 mmol 1⁻¹; 1 mCi mmol⁻¹) and inulin (0.2 µCi per ml medium) were added. Muscles were blotted and frozen in liquid nitrogen 20 min after addition of 2-DG. Muscles were then weighed, digested in NaOH at 70°C, cooled and centrifuged, and aliquots of the supernatants were added to scintillation cocktail and counted for ³H (2-DG) and ¹⁴C (inulin).

In some experiments on EDL muscles, 2-DG glucose uptake was measured after 2-APB removal in the continued presence of insulin. After the initial 30 min incubation period, insulin (10 milliunits ml⁻¹) was added and remained in the solution for the rest of the experiment. After an additional 30 min, 2-APB (100 µM) was added. Thirty min later muscles were transferred to medium that did not contain 2-APB and after an additional 10 min, muscles were exposed to 2-DG (as above) for 20 min before being blotted and frozen. Control muscles were treated in the same way except that 2-APB was never added.

The effect of 2-APB on contraction-mediated 2-DG uptake was studied in EDL muscles. The muscles were first stimulated by two tetanic contractions (50 Hz, 100 ms duration) per sec for 10 min. This resulted in a decrease in the glycogen store to less than 20% of the rested value (data not shown). Muscles were allowed to rest for 20 min after the stimulation period and were then exposed to 2-DG for 20 min before being blotted and frozen (as described above). Some muscles were exposed to 2-APB (100 µM) throughout the 40 min recovery period after stimulation.

Experiments on single FDB muscle fibers were performed at 24 °C. The isolated fiber was mounted in a stimulation chamber and continuously superfused by standard Tyrode solution (Lännergren et al. (1987) J Physiol 390:285-293). The free myoplasmic [Ca²⁺] ([Ca²⁺]_{¡}) was measured with the fluorescent Ca²⁺ indicator indo-1 (Andrade et al. (1998) J Physiol 509:565-575). An indirect measure of SMCE was obtained by measuring the rate of Mn²⁺ entry and quenching of the fluorescence intensity of fura-2 (Hopfet al. (1996) J Biol Chem 271:22358-22367). MnCl₂ (100 µmol 1⁻¹) was added to the bath solution and the fura-2 injected muscle fiber was excited at 360 ± 5 nm (the isosbestic wavelength of fura-2 under our experimental conditions) while measuring the fluorescence light emitted at 495 ± 5 nm. The rate of Mn²⁺ quenching was measured during periods where the rate of decrease of the fluorescent light was stable, i.e. ignoring transient changes induced by changes of the bath solution. Measurements were not performed until 2 min after application of insulin, thus allowing time for insulin to act.

Data are presented as mean ± SEM. Statistical analyses were performed as paired or unpaired *t* tests or one-way analysis of variance followed by the Student-Newman-Keuls multiple comparison test. *P* values < 0.05 were considered statistically significant.

### EXAMPLES

### EXAMPLE 1: Pharmacological inhibition of SMCE decreases insulin-mediated 2-DG uptake

Application of 2-APB decreased insulin-mediated 2-DG uptake in a dose-dependent manner both in EDL and soleus muscles, although the slow-twitch soleus muscles appeared somewhat more sensitive (Fig. 1A). To ensure that this inhibition was not caused by a generalized, unspecific inhibitory effect of the drug, contractile function was tested during exposure to 2-APB. After 30 min exposure to 100 µmol 1⁻¹ 2-APB, tetanic force was increased by 11 ± 3% (n = 5; P < 0.05) and the relaxation speed was slightly decreased; thus, cells were able to generate action potentials in response to electrical stimulation and their contractile machinery was intact. The increase in tetanic force combined with slowed relaxation mimics the situation when the ATP-driven SR Ca²⁺ uptake is inhibited (Westerblad et al. (1994) J Physiol 474:291-301). To ensure that the effect of 2-APB on 2-DG glucose uptake was not due to inhibition of SR Ca²⁺ uptake, we performed control experiments with TBQ. Application of TBQ (500 nmol 1⁻¹) resulted in an increase of tetanic force by 4 ± 2% (n = 4; change in force not significantly different from that with 2-APB) and a slight slowing of relaxation. TBQ had no effect on insulin-mediated 2-DG uptake. In soleus muscles insulin-mediated uptake was 163 ± 8 (n = 6), 150 ± 7 (n = 6) and 179 ± 8 (n = 4) µmol 1⁻¹ min⁻¹ without and with 100 nmol 1⁻¹ and 500 nmol 1⁻¹ TBQ, respectively. Corresponding values in EDL muscles were 89 ± 5 (n = 6), 93 ± 5 (n = 6) and 103 ± 4 (n = 4) µmol 1⁻¹ min⁻¹, respectively.

Application of MDL, another SMCE inhibitor, also decreased insulin-mediated 2-DG uptake in a dose-dependent manner both in EDL and soleus muscles (Fig. 1B). As with 2-APB, soleus muscles appeared more sensitive to MDL than EDL muscles. However in contrast to 2-APB, application of 100 µmol 1⁻¹ MDL resulted in a gradual reduction of tetanic force and after 30 min of exposure force was reduced by 22 ± 2% (n = 4; P < 0.01). This reduction was not reversed on washout of the drug.

Mean data for basal 2-DG uptake (i.e. in the absence of insulin) are presented in Table I. Contrary to the insulin-mediated 2-DG uptake, the basal uptake was not significantly affected by application of 2-APB or MDL. TBQ also had no significant effect on the basal DG uptake. Thus, inhibition of SMCE markedly reduced insulin-mediated 2-DG uptake, whereas the uptake under basal conditions was not affected.

**TABLE I: Basal rate of 2-DG uptake**

| | EDL | Soleus |
|---|---|---|
| Control | 32 ± 2 (21) | 48 ± 3 (20) |
| 2-APB (100 or 1000 µmol 1⁻¹) | 40 ± 4 (7) | 55 ± 3 (6) |
| MDL (300 µmol 1⁻¹) | 42 ± 3 (4) | 42 ± 4 (4) |
| TBQ (500 nmol 1⁻¹) | 37 ± 6 (4) | 63 ± 5 (4) |

| | | |
|---|---|---|
| Data (µmol 1⁻¹ min⁻¹) are means ± SEM (n). The concentration of 2-APB was 0.1 mmol 1⁻¹ for EDL and 1.0 mmol 1⁻¹ for soleus. | | |

### EXAMPLE 2: Pharmacological inhibition of SMCE decreases the rate of Mn²⁺ quenching

The rate of Mn²⁺ quenching of fura-2 fluorescence was used to assess changes in SMCE activity associated with application of insulin and 2-APB. After addition of Mn²⁺ to the standard Tyrode solution (containing 5.5 mmol 1⁻¹ glucose), the rate of quenching (expressed as per cent of the initial fluorescence intensity) was 0.72 ± 0.05 % min⁻¹ (n = 12). Figure 2A shows original records of the fura-2 fluorescence during a quenching experiment where a single muscle fiber was exposed to 2-APB (100 µmol 1⁻¹) and subsequently to insulin (10 mU ml⁻¹) in the continued presence of 2-APB. It can be seen that the rate of quenching was markedly reduced when 2-APB was added and partially recovered after application of insulin. Similar results were obtained in five more experiments and mean data are shown in Fig. 2B. Thus, application of 2-APB decreased the rate of quenching by about 70% and insulin restored about half of this decrease. The marked decrease of the rate of quenching with 2-APB application would reflect a proportional reduction in the rate of Ca²⁺ influx into the cells, which might affect [Ca²⁺]_{¡}. Therefore, we measured changes in global [Ca²⁺]_{¡} (using indo-1) in association with 2-APB exposure. Application of 2-APB caused a clear decrease of [Ca²⁺]_{¡} from 70 ± 10 to 51 ± 9 nmol l⁻¹ (n = 5; *P* < 0.01).

Another series of quenching experiments was performed in which insulin was first applied and then 2-APB was added. Figure 3A shows original records from one such experiment where insulin exposure resulted in a slight increase in the rate of quenching. An increased rate of quenching was observed in one more fiber, whereas the other four fibers tested showed a decrease or no change at all. Thus, mean data showed no significant insulin-induced change of the rate of quenching (Fig. 3B, filled circles). The subsequent exposure to 2-APB in the presence of insulin resulted in a marked reduction of the rate of quenching. Interestingly, removal of 2-APB in the continued presence of insulin resulted in a rate of quenching that was significantly (about 20%; *P* < 0.05) higher than the original. [Ca²⁺]_{¡} was measured in a separate series of experiments (Fig. 3C). Mean data (n = 4) showed no change of global [Ca²⁺]_{¡} when insulin was applied (75 ± 13 *vs.* 74 ± 13 nmol 1⁻¹), whereas the subsequent application of 2-APB resulted in a rapid and significant (*P* < 0.05) decrease of [Ca²⁺]_{¡} (56 ± 9 nmol l⁻¹) that was reversed on washout of insulin and 2-APB (76 ± 12 nmol 1⁻¹).

Since all quenching experiments described thus far experiments were performed with glucose in the incubation buffer, a series of Mn²⁺ quenching experiments was performed in Tyrode solution with 0 mmol 1⁻¹ glucose. The basal rate of quenching in zero glucose (0.64 ± 0.06% min⁻¹; n = 5) was not significantly different from that in 5.5 mmol 1⁻¹ glucose (see above). On the other hand, addition of insulin in zero glucose resulted in a small (about 10%) increase in the rate of quenching in all fibers tested (Fig. 3B; unfilled circles). The response to addition and removal of 2-APB was qualitatively similar in zero and 5.5 mmol 1⁻¹ glucose, although the rate of quenching was generally higher in the former. The reason for this difference was not further investigated.

The contractile response to tetanic stimulation (70 Hz, 350 ms duration) was tested before and after exposures to 2-APB in most of the above Mn²⁺ quenching experiments and neither tetanic [Ca²⁺]_{¡} nor force showed any noticeable changes (data not shown).

Removal of 2-APB in the continued presence of insulin results in increased 2-DG uptake. Fig. 3B shows that the rate of Mn²⁺ quenching is increased after removal of 2-APB. Having this in mind, we measured the rate of 2-DG uptake in EDL muscles under similar experimental conditions. The results showed a marked (∼30%) increase in 2-DG uptake in muscles previously exposed to 2-APB and insulin as compared to insulin alone (Fig. 4).

### EXAMPLE 3: 2-APB exposure does not affect contraction-mediated 2-DG uptake

In addition to insulin, repeated contractions induce a marked increase in glucose uptake in skeletal muscle. To test if 2-APB also affected contraction-mediated 2-DG uptake, EDL muscles were exposed to intense, repeated tetanic stimulation for 10 min and then allowed to recover in the presence or absence of 2-APB. The stimulation protocol decreased force production to ∼10% of the original (Fig. 5A) and increased 2-DG uptake about 3-fold both in the presence and absence of 2-APB (Fig. 5B).

Thus, SMCE appears not to be involved in this alternative way to activate glucose transport. It should be noted that repeated contractions as such result in a marked increase in [Ca²⁺]ᵢ. This increase may be sufficient to support glucose transport and hence activation of SMCE would not be required.

### EXAMPLE 4: Identification of agents modulating glucose uptake

Agents modulating the activity of SMCE and the rate of glucose uptake in human cells can be identified by methods known in the art, e.g. by any of the following methods:

### Quenching assay

This assay will indirectly measure the rate of Ca²⁺ influx by measuring the rate of Mn²⁺-quenching of fura-2 fluorescence (cf. Example 2). Briefly, cultured cells are loaded with fura-2AM in the standard fashion (e.g., Basavappa et al. (1999) Biochem Biophys Res Comm 254:699-702). After loading they are allowed to rest for about 1 hour to make sure that all fura-AM has been converted to the active form. MnCl₂ (100-500 µM) is then added to the bath solution while measuring the rate of quenching (decline) of fura-2 fluorescence (excitation and emission wavelengths as in Example 2) for about 10 min. A drug candidate is then added to the bath +/- insulin, and possible changes in the rate of Mn²⁺ quenching are measured. Increased quenching suggests increased Ca²⁺ influx, which may reflect increased glucose uptake.

### Assay based on genetically-encoded fluorescent Ca²⁺ indicators

In this assay Ca²⁺ is measured with a cameleon, i.e. a fluorescent Ca²⁺ indicator protein. A cameleon consists of a cyan fluorescent protein, calmodulin (CaM), a glycylglycine linker, the CaM-binding domain of myosin light chain kinase, and a yellow fluorescent protein. Ca²⁺ binding to CaM results in a change of the conformation of this molecule and hence altered fluorescence. The cameleons can be targeted to specific intracellular sites by fusing them to specific host proteins (e.g. Miyawaki et al. (1997) Nature 388: 882-887). In this assay the cameleon will initially be fused to some surface membrane protein. Ultimately, it will be fused to the target that is the likely candidate to be activated by insulin.

Cultured cells are infected by adenoviral vectors of the cameleon YC2.1 as described in Miyawaki et al. (1999) Proc. Natl. Acad. Sci. U.S.A. 96: 2135-2140. The vectors are modified so that they will fuse with a suitable surface membrane host protein (see above). Ca²⁺ concentration is measured by the cells being excited at 432 nm while measuring the light emitted at 535 nm and 480 nm. Increases in the 535 nm / 480 nm ratio reflects increased Ca²⁺ concentration. To obtain a qualitative measure of changes in Ca²⁺ concentration (i.e. it goes up or down), it would be possible to only measure at one of the above emission wavelengths. Ca²⁺ concentration will be measured under control conditions and after addition of a drug candidate +/- insulin.

## Claims

1. A method for identifying an agent that modulates glucose uptake in a mammalian cell, the method comprising:
contacting a mammalian cell with a candidate agent;
determining whether the candidate agent modulates store-mediated Ca²⁺ entry (SMCE) into the cell; and
determining whether the candidate agent modulates glucose uptake in the cell.

2. The method of claim 1, wherein the method comprises determining whether the candidate agent increases SMCE into the cell.

3. The method of claim 2, wherein the method comprises determining whether the candidate agent increases glucose uptake in the mammalian cell.

4. A method for identifying an agent that modulates glucose uptake in a mammalian cell, the method comprising:
contacting a mammalian cell with a candidate agent, wherein the candidate agent modulates SMCE into the cell;
measuring glucose uptake in the cell in the presence of the candidate agent; and
determining whether the candidate agent modulates glucose uptake in the cell, wherein altered glucose uptake in the cell in the presence of the candidate agent compared to the absence of the candidate agent indicates that the candidate agent modulates glucose uptake.

5. The method of claim 4, wherein the candidate agent increases SMCE into the cell and thereby increases glucose uptake in the cell.

## Patentansprüche

1. Verfahren zum Identifizieren eines Wirkstoffs, der die Glukoseaufnahme in eine Säugetierzelle moduliert, wobei das Verfahren umfasst:
Kontaktieren einer Säugetierzelle mit einem Wirkstoffkandidaten;
Bestimmen, ob der Wirkstoffkandidat den Speicher-vermittelten Ca²⁺-Eintritt (SMCE) in die Zelle moduliert; und
Bestimmen, ob der Wirkstoffkandidat die Glukoseaufnahme in die Zelle moduliert.

2. Verfahren nach Anspruch 1, wobei das Verfahren das Bestimmen umfasst, ob der Wirkstoffkandidat SMCE in die Zelle erhöht.

3. Verfahren nach Anspruch 2, wobei das Verfahren das Bestimmen umfasst, ob der Wirkstoffkandidat die Glukoseaufnahme in die Säugetierzelle erhöht.

4. Verfahren zum Identifizieren eines Wirkstoffs, der die Glukoseaufnahme in eine Säugetierzelle moduliert, wobei das Verfahren umfasst:
Kontaktieren einer Säugetierzelle mit einem Wirkstoffkandidaten, wobei der Wirkstoffkandidat SMCE in die Zelle moduliert;
Messen der Glukoseaufnahme in die Zelle in Gegenwart des Wirkstoffkandidaten; und
Bestimmen, ob der Wirkstoffkandidat die Glukoseaufnahme in die Zelle moduliert, wobei eine geänderte Glukoseaufnahme in die Zelle in Gegenwart des Wirkstoffkandidaten im Vergleich zur Abwesenheit des Wirkstoffkandidaten anzeigt, dass der Wirkstoffkandidat die Glukoseaufnahme moduliert.

5. Verfahren nach Anspruch 4, wobei der Wirkstoffkandidat SMCE in die Zelle erhöht und **dadurch** die Glukoseaufnahme in die Zelle erhöht.

## Revendications

1. Procédé d'identification d'un agent qui module l'assimilation de glucose dans une cellule de mammifère, le procédé comprenant les étapes consistant à :
mettre en contact une cellule de mammifère avec un agent potentiel ;
déterminer si l'agent potentiel module l'entrée Ca2+ facilitée (SMCE) à l'intérieur de la cellule ; et
déterminer si l'agent potentiel module l'assimilation de glucose dans la cellule.

2. Procédé selon la revendication 1, dans lequel le procédé comprend l'étape consistant à déterminer si l'agent potentiel augmente la SMCE à l'intérieur de la cellule.

3. Procédé selon la revendication 2, dans lequel le procédé comprend l'étape consistant à déterminer si l'agent potentiel augmente l'assimilation de glucose dans la cellule de mammifère.

4. Procédé d'identification d'un agent qui module l'assimilation de glucose dans une cellule de mammifère, le procédé comprenant les étapes consistant à :
mettre en contact une cellule de mammifère avec un agent potentiel, dans lequel l'agent potentiel module la SMCE à l'intérieur de la cellule ;
mesurer l'assimilation de glucose dans la cellule en présence de l'agent potentiel ; et déterminer si l'agent potentiel module l'assimilation de glucose dans la cellule, dans lequel l'assimilation du glucose altéré dans la cellule en présence de l'agent potentiel en comparaison de l'absence de l'agent potentiel indique que l'agent potentiel module l'assimilation de glucose.

5. Procédé selon la revendication 4, dans lequel l'agent potentiel augmente la SMCE à l'intérieur de la cellule et augmente de ce fait l'assimilation de glucose dans la cellule.
